# EUROPÄISCHE PATENTSCHRIFT

(11) **EP 3 876 239 B1**
(45) Veröffentlichungstag und Bekanntmachung des Hinweises auf die Patenterteilung: **01.05.2024**
(21) Anmeldenummer: 20160636.5
(22) Anmeldetag: 03.03.2020
(51) Int. Cl.: G16H 40/40, A61C 1/02

(54) **VERFAHREN ZUR DRAHTLOSEN ÜBERTRAGUNG VON DATEN IN EINEM MEDIZINISCHEN ODER DENTALEN SYSTEM UND DERARTIGES MEDIZINISCHES ODER DENTALES SYSTEM**
METHOD FOR WIRELESS TRANSMISSION OF DATA IN A MEDICAL OR DENTAL SYSTEM AND SUCH MEDICAL OR DENTAL SYSTEM
PROCÉDÉ DE TRANSMISSION SANS FIL DE DONNÉES DANS UN SYSTÈME MÉDICAL OU DENTAIRE AINSI QU'UN TEL SYSTÈME MÉDICAL OU DENTAIRE

(43) Veröffentlichungstag der Anmeldung: 08.09.2021
(73) Patentinhaber: W & H Dentalwerk Bürmoos GmbH, 5111 Bürmoos (AT)
(72) Erfinder: SILBERER, Bernhard, 5152 Michaelbeuern (AT); REITER, Michael, 5061 Elsbethen (AT)
(74) Vertreter: Benda, Ralf

(56) Entgegenhaltungen:
- US-A1- 2019 201 136
- Anonymous: "Quality of service - Wikipedia", , 16. Februar 2019 (2019-02-16), XP055691561, Gefunden im Internet: URL:https://en.wikipedia.org/w/index.php?t itle=Quality_of_service&oldid=883636447 [gefunden am 2020-05-05]
- Anonymous: "Traffic shaping - Wikipedia", , 14. Juni 2016 (2016-06-14), XP055635406, Gefunden im Internet: URL:https://en.wikipedia.org/w/index.php?t itle=Traffic_shaping&oldid=725195597 [gefunden am 2019-10-23]

## Beschreibung

Die vorliegende Erfindung betrifft ein Verfahren zur drahtlosen Übertragung von Daten in einem medizinischen oder dentalen System und ein derartiges medizinisches oder dentales System mit einer Fußsteuerung und mehreren medizinischen oder dentalen Geräten zur Behandlung und/ oder Diagnose, wobei zumindest zwei der mehreren Geräte wahlweise durch die Fußsteuerung ansteuerbar sind.

Aus der Patentanmeldung US 2005/0251228 A1 ist eine Fußsteuerung bekannt, die ausgebildet ist, wahlweise eines von mehreren medizinischen Geräten drahtlos zu steuern. Die Auswahl, an welches Gerät drahtlos Daten von der Fußsteuerung übertragen werden soll, trifft der Anwender durch ein Stellelement an der Fußsteuerung. Eine derartige Auswahl des Geräts über ein Stellelement an der Fußsteuerung ist für den Anwender unpraktisch, gegebenenfalls aufgrund des Schuhwerks beschwerlich und beinhaltet somit viele Fehlermöglichkeiten.

Auch in Ausführungsbeispielen der Patentanmeldung US 2019/0201136 A1 ist eine Fußsteuerung vorgesehen, durch die mehrere medizinische Geräte gesteuert werden können. Die Auswahl, welches dieser medizinischen Geräte tatsächlich angesteuert wird, trifft der Anwender, zum Beispiel durch Betätigung eines Stellelements an der Fußsteuerung, durch Sprachsteuerung oder über eine grafische Benutzeroberfläche.

Auf der Internetseite "Quality of service" (Quelle: https://en.wikipedia.org/w/index.php ?title=Quality_of_service&oldid=883636447) ist offenbart, dass sich im Bereich der Computernetzwerke und anderer paketvermittelter Telekommunikationsnetze die Dienstqualität eher auf Mechanismen zur Priorisierung des Datenverkehrs und zur Steuerung der Ressourcenreservierung als auf die erreichte Dienstqualität bezieht. Dienstqualität ist die Fähigkeit, verschiedenen Anwendungen, Benutzern oder Datenströmen unterschiedliche Prioritäten zuzuweisen oder einem Datenstrom ein bestimmtes Leistungsniveau zu garantieren.

Der vorliegenden Erfindung liegt daher die Aufgabe zugrunde Verfahren und entsprechende Vorrichtungen oder Systeme zu schaffen, bei denen die Auswahl, an welches Gerät drahtlos Daten von der Fußsteuerung übertragen werden soll, für den Anwender einfacher zu bewerkstelligen ist und die Möglichkeiten einer Fehlbedienung verringert sind.

Diese Aufgabe wird gemäß der vorliegenden Erfindung durch ein Verfahren zur drahtlosen Übertragung von Daten in einem medizinischen oder dentalen System mit den Merkmalen des Anspruchs 1, ein Computerprogrammprodukt oder computerlesbares Speichermedium gemäß Anspruch 11, eine Fußsteuerung mit den Merkmalen des Anspruchs 12 und ein medizinisches oder dentales System mit den Merkmalen des Anspruchs 14 gelöst. Besonders vorteilhafte Ausführungsformen sind in den jeweiligen Unteransprüchen angeführt. Der Schutzumfang der vorliegenden Erfindung ist durch diese Patentansprüche definiert.

Zur Durchführung des erfindungsgemäßen Verfahrens ist ein medizinisches oder dentales System vorgesehen, das eine Fußsteuerung und mehrere medizinische oder dentale Geräte, zum Beispiel ein Handstück oder ein Tischgerät mit Handstück, zur Behandlung und/ oder Diagnose umfasst. Zumindest zwei der mehreren Geräte sind wahlweise durch die Fußsteuerung drahtlos ansteuerbar, wobei jedem durch die Fußsteuerung ansteuerbaren Gerät ein spezifischer, das heißt insbesondere ein individueller und/ oder eindeutiger und/ oder nicht veränderbarer, Identifikationscode zugeordnet ist. Das erfindungsgemäße Verfahren zur drahtlosen Übertragung von Daten in dem medizinischen oder dentalen System ist dadurch definiert, dass eine in der Fußsteuerung vorgesehenen Steuerlogik auf Basis von Datenpaketen jenes durch die Fußsteuerung ansteuerbare Gerät auswählt, an das drahtlos Betriebsdaten, insbesondere Steuerungsdaten für die Behandlung und/ oder Diagnose übertragen werden, wobei jedem durch die Fußsteuerung ansteuerbaren Gerät ein eigenes Datenpaket zugeordnet ist. Jedes dieser Datenpakete umfasst (1) den spezifischen Identifikationscode des durch die Fußsteuerung ansteuerbaren Geräts, dem das Datenpaket zugeordnet ist, und (2) einen diesem Gerät (dem das Datenpaket zugeordnet ist) zugeordneten, individuellen Rang einer Rangordnung der durch die Fußsteuerung ansteuerbaren Gerät. Ein Datenpaket ist also einem durch die Fußsteuerung ansteuerbaren Gerät zugeordnet und umfasst ausschließlich dessen spezifischen Identifikationscode und dessen einmaligen, individuellen Rang. Ein spezifischer Identifikationscode und ein einmaliger, individueller Rang bilden somit vorzugsweise eine untrennbare Einheit in Form des Datenpakets, die einem bestimmten Gerät zugeordnet ist.

Wie im Folgenden noch im Detail beschrieben wählt die in der Fußsteuerung vorgesehenen Steuerlogik somit auf Basis einer vorbestimmten Rangordnung, gemäß der jedem durch die Fußsteuerung ansteuerbaren Gerät ein vorbestimmter, individueller und in dem jeweiligen Datenpaket enthaltener Rang zugeordnet ist, jenes Gerät aus, an welches (aktuell oder im Folgenden) drahtlos Betriebsdaten oder Steuerungsdaten für die Behandlung und/ oder Diagnose übertragen werden.

Somit entfällt in vorteilhafter Weise die für den Anwender beschwerliche und fehleranfällige Auswahl des anzusteuernden Instruments über die Fußsteuerung.

Die drahtlose Übertagung der Daten umfasst insbesondere Funkübertragung im Frequenzbereich von Radiowellen, zum Beispiel durch Bluetooth oder WLAN. Auch Datenübertragung mit Wellenlängen im Infrarotbereich ist denkbar.

Der spezifische Identifikationscode, der jedem durch die Fußsteuerung ansteuerbaren Gerät, zum Beispiel einem Handstück, zugeordnet ist, umfasst zum Beispiel eine numerische oder alphanumerische Zeichenkette, einen PIN-Code, eine Geräteadresse oder eine MAC-Adresse. Vorzugsweise umfasst jedes der durch die Fußsteuerung ansteuerbaren Geräte ein Speicherelement, in dem der jeweilige dem Gerät zugeordnete, spezifische Identifikationscode gespeichert ist. Dieses Speicherelement und/ oder der Identifikationscode sind für den Anwender vorzugsweise nicht überschreibbar und/ oder löschbar. Damit ist in vorteilhafter Weise eine eindeutige Identifikation oder Zuordnung des Geräts möglich, insbesondere durch die Fußsteuerung oder Steuerlogik, welche den Identifikationscode empfängt oder zu dessen Empfang ausgebildet ist.

Vorzugsweise sind die durch die Fußsteuerung ansteuerbaren Geräte ausgebildet, ihren jeweiligen spezifischen Identifikationscode oder ihr jeweiliges Datenpaket drahtlos an die gemeinsame Fußsteuerung zu senden, welche zur Steuerung des Geräts ausgebildet ist. Insbesondere wenn die Fußsteuerung einen spezifischen Identifikationscode, vorzugsweise als Teil des Datenpakets, empfängt, ist für die Fußsteuerung somit erkennbar, ob ein (bestimmtes) oder welches durch die Fußsteuerung ansteuerbares Gerät (aktuell) aktiv ist, zum Beispiel eingeschaltet ist oder sich in einem Betriebs- oder Aktivitätsmodus oder in Betrieb befindet, und/ oder ob ein (bestimmtes) oder welches durch die Fußsteuerung ansteuerbares Gerät (aktuell) für eine Kommunikation oder Datenübertragung mit der Fußsteuerung bereit ist. Entsprechend ist, wenn die Fußsteuerung einen spezifischen Identifikationscode oder ein spezifisches Datenpaket nicht empfängt, für die Fußsteuerung somit in vorteilhafter Weise erkennbar, dass ein bestimmtes oder welches durch die Fußsteuerung ansteuerbares Gerät (aktuell) nicht aktiv ist, zum Beispiel nicht eingeschaltet ist oder sich nicht in einem Betriebs- oder Aktivitätsmodus oder in Betrieb befindet, und/ oder dass ein bestimmtes oder welches durch die Fußsteuerung ansteuerbares Gerät (aktuell) nicht für eine Kommunikation oder Datenübertragung mit der Fußsteuerung bereit ist.

Die in der Fußsteuerung vorgesehenen Steuerlogik, die zum Empfang der Identifikationscodes oder der Datenpakete ausgebildet ist, umfasst insbesondere elektronische Schaltungen und/ oder Software. Die Steuerlogik ist ausgebildet, empfangene Identifikationscodes oder Datenpakete zu verarbeiten und/ oder mit in der Fußsteuerung gespeicherten Identifikationscodes oder Datenpakete (der durch die Fußsteuerung ansteuerbaren Geräte) zu vergleichen und/ oder auszuwerten, um jenes Gerät auszuwählen, an welches drahtlos Betriebsdaten oder Steuerungsdaten für die Behandlung und/ oder Diagnose übertragen werden. Damit wird dem Anmelder in vorteilhafter Weise eine sehr bedienungsfreundliche Möglichkeit geboten das durch die Fußsteuerung anzusteuernde Gerät zu bestimmen.

Die Auswahl des Geräts, an welches (aktuell) drahtlos Betriebsdaten oder Steuerungsdaten für die Behandlung und/ oder Diagnose übertragen werden, durch die Steuerlogik erfolgt auf Basis einer (vorbestimmten) Rangordnung der durch die Fußsteuerung ansteuerbaren Geräte. Vorzugsweise umfasst die Rangordnung eine Rangfolge, die insbesondere in einfacher Weise für oder mit Hilfe der Steuerlogik unterscheidbar ist. In der Rangordnung ist jedem durch die Fußsteuerung ansteuerbaren Gerät ein individueller und/ oder eindeutiger und/ oder eigener und/ oder vorbestimmter Rang zugeordnet, wobei jeder Rang in der Rangordnung nur einmal vorkommt und somit insbesondere jedes Datenpaket einen eigenen, eindeutigen oder einmaligen Rang aufweist. Die Ränge weisen vorzugsweise unterschiedliche numerische Werte auf, zum Beispiel ist ein Rang mit dem Wert 1, ein weiterer Rang mit dem Wert 2, ein weiterer Rang mit dem Wert 3 usw. vorgesehen. Selbstverständlich sind auch Rangordnungen mit anderen Werten möglich, zum Beispiel mit unterschiedlich langen Zeichenketten. Die Rangfolge kann zum Beispiel in Form einer Liste ausgebildet sein. Damit ist in vorteilhafter Weise eine sowohl in Bezug auf Hardware als auch auf Software äußerst einfach zu realisierende Möglichkeit zur Auswahl des anzusteuernden Geräts geschaffen.

Gemäß einem bevorzugten Ausführungsbeispiel der Erfindung sind die Datenpakete der durch die Fußsteuerung ansteuerbaren Geräte in der Fußsteuerung vorgesehen, insbesondere gespeichert. Damit sind die Datenpakete in vorteilhafter Weise zentral gespeichert und damit zum Beispiel einfach anzupassen oder zu ergänzen. Die Datenpakete sind insbesondere in einem Speicherelement der Fußsteuerung gespeichert, wobei das Speicherelement vorzugsweise mit der Steuerlogik kommunikativ verbunden ist oder als Teil der Speicherlogik ausgebildet ist. Vorzugsweise speichert die Fußsteuerung zumindest zwei spezifische Datenpakete von zwei Geräten und entsprechend zwei individuelle Ränge, zum Beispiel einen Identifikationscode AAA eines ersten Geräts, dem ein Rang 1 zugeordnet ist, und einen Identifikationscode BBB eines zweiten Geräts, dem ein Rang 2 zugeordnet ist. Selbstverständlich können weitere Datenpakete mit spezifischen, individuellen Identifikationscodes für zusätzliche Geräte, zum Beispiel CCC, DDD, EEE,... und weiteren jeweils einem der Geräte zugeordneten Rängen 3, 4, 5,... vorhanden sein.

Vorzugsweise senden (an einem bestimmten Zeitpunkt) eines oder mehrere der durch die Fußsteuerung ansteuerbaren Geräte ihren jeweiligen im Gerät gespeicherten, spezifischen Identifikationscode an die Fußsteuerung, wobei die Fußsteuerung diese(n) spezifischen Identifikationscode(s) empfängt, vorzugsweise speichert oder zwischenspeichert, und die Steuerlogik auf Basis des oder der empfangenen Identifikationscode(s) und der (in der Fußsteuerung gespeicherten) Datenpakete erkennt, welche(s) der durch die Fußsteuerung ansteuerbaren Geräte aktiv ist/ sind und welchen individuellen Rang das oder die aktive(n) Gerät(e) in der Rangordnung einnimmt/ einnehmen. Besonders bevorzugt vergleicht die Steuerlogik jeden von einem Gerät gesendeten und empfangenen Identifikationscode mit den in den Datenpaketen vorgesehenen Identifikationscodes und erkennt daraus, welches der mehreren durch die Fußsteuerung ansteuerbaren Geräte (aktuell) aktiv ist, zum Beispiel eingeschaltet ist oder sich in einem Betriebs- oder Aktivitätsmodus oder in Betrieb befindet, und somit grundsätzlich für eine Kommunikation oder Datenübertragung mit der Fußsteuerung bereit ist.

Durch den Vergleich des gesendeten und empfangenen Identifikationscodes mit den in den Datenpaketen vorgesehenen Identifikationscodes kann die Steuerlogik auch den individuellen Rang jedes aktiven Geräts bestimmen, da gemäß der Rangordnung jedem durch die Fußsteuerung ansteuerbaren Gerät, d.h. dessen Identifikationscode ein individuellen Rang zugeordnet ist. Damit ist in vorteilhafter Weise eine sehr zuverlässige Vorgehensweise zur Ermittlung der aktiven Geräte und insbesondere deren jeweiligen individuellen Ränge geschaffen.

Das Verfahren ist so beschaffen, dass, wenn die Fußsteuerung (an einem bestimmten Zeitpunkt oder innerhalb eines begrenzten Zeitraums) spezifische Identifikationscodes von zwei oder mehr durch die Fußsteuerung ansteuerbaren Geräten empfängt, dann wählt die Fußsteuerung - insbesondere basierend auf dem im Vorstehenden beschriebenen Vergleich des gesendeten und empfangenen Identifikationscode mit den in den Datenpaketen vorgesehenen Identifikationscodes und der daraus ermittelten Ränge der aktiven Geräte - jenes Gerät zur drahtlosen Übertragung von Betriebsdaten oder Steuerungsdaten für die Behandlung und/ oder Diagnose aus, von dem die Fußsteuerung einen Identifikationscode empfangen hat und das in der Rangordnung einen im Vergleich zu den übrigen Geräten, von denen die Fußsteuerung einen Identifikationscode empfangen hat, priorisierten individuellen Rang einnimmt oder Wert aufweist. Der priorisierte Rang kann im Vergleich zu den anderen individuellen Rängen der aktiven Geräte zum Beispiel eine bevorzugte oder übergeordnete oder höhere oder niedrigere Position in der Rangordnung oder in einer Rangliste oder einen bevorzugten oder übergeordneten oder höheren oder niedrigeren Wert aufweisen. Damit ist in vorteilhafter Weise eine einfache und zuverlässige Vorgehensweise zur drahtlosen Übertragung von Betriebsdaten geschaffen.

Gemäß einer Ausführungsform der Erfindung umfasst das medizinische oder dentale System exakt zwei Geräte, die mit der Fußsteuerung steuerbar sind. Diesen zwei Geräten ist jeweils ein spezifischer Identifikationscode zugeordnet, zum Beispiel WHTW und WHUL, die in jeweiligen Speicherelementen der Geräte gespeichert sind. Sind beide Geräte aktiv und senden beide Geräte (an einem bestimmten Zeitpunkt oder während eines begrenzten Zeitraums) gleichzeitig ihren jeweiligen, spezifischen Identifikationscode und empfängt die (gemeinsame) Fußsteuerung beide Identifikationscodes, so vergleicht die Fußsteuerung die beiden empfangenen Identifikationscodes mit den Identifikationscodes der gespeicherten Datenpakete und ermittelt damit auch den jeweiligen individuellen Rang, der dem jeweiligen Identifikationscode in der Rangordnung zugeordnet ist. Zum Beispiel ist dem Identifikationscode WHTW der individuelle Rang 0 und dem Identifikationscode WHUL der individuelle Rang 1 zugeordnet. Ist die Steuerlogik zum Beispiel derart ausgebildet, dass der niedrigste individuelle Rang oder Wert der Rangordnung priorisiert ist, so baut die Fußsteuerung eine drahtlose Kommunikationsverbindung zur Übertragung von Betriebsdaten oder Steuerungsdaten für die Behandlung und/ oder Diagnose nur zu dem Gerät mit dem Identifikationscode WHTW (mit dem individuellen Rang 0) auf. Entsprechend diesem Ausführungsbeispiel baut die Fußsteuerung somit keine drahtlose Kommunikationsverbindung zur Übertragung von Betriebsdaten zu dem Gerät mit dem Identifikationscode WHUL (mit dem individuellen Rang 1) auf.

Das im Vorstehenden beschriebene Ausführungsbeispiel gilt entsprechend für medizinische oder dentale System mit mehr als zwei Geräten, die mit der Fußsteuerung steuerbar sind, und bei denen (an einem bestimmten Zeitpunkt oder während eines begrenzten Zeitraums) mehr als zwei Geräte gleichzeitig ihren jeweiligen Identifikationscode senden. Wiederum empfängt und vergleicht die Steuerlogik die mehr als zwei spezifischen Identifikationscodes der mehr als zwei aktiven Geräte mit den Identifikationscodes der Datenpakete und ermittelt damit auch die jeweiligen individuellen Ränge der mehr als zwei aktiven Geräte. Basierend auf den ermittelten individuellen Rängen baut die Fußsteuerung eine drahtlose Kommunikationsverbindung zur Übertragung von Betriebsdaten oder Steuerungsdaten für die Behandlung und/ oder Diagnose nur zu jenem Gerät auf, dessen individueller Rang priorisiert ist.

Gemäß einem alternativen Ausführungsbeispiel der Erfindung weist jedes durch die Fußsteuerung ansteuerbares Gerät sein ihm zugeordnetes Datenpaket auf. Die Datenpakete der durch die Fußsteuerung ansteuerbaren Geräte sind somit vorzugsweise in dem jeweiligen Gerät vorgesehen, insbesondere gespeichert, dessen spezifischen Identifikationscode und einmaligen, individuellen Rang es enthält. Jedes Datenpaket kann individuell von ,seinem` durch die Fußsteuerung ansteuerbaren Geräte (drahtlos) an die Fußsteuerung übertragen werden. Damit können in vorteilhafter Weise sehr einfach zusätzliche Geräte in dem Verfahren und System ergänzt werden, insbesondere ohne Anpassungen oder Ergänzungen an der Fußsteuerung. Das jeweilige Datenpaket ist vorzugsweise in einem Speicherelement des jeweiligen Geräts gespeichert.

Vorzugsweise ist das Verfahren so beschaffen, dass, wenn die Fußsteuerung Datenpakete von zwei oder mehr durch die Fußsteuerung ansteuerbaren Geräten empfängt, dann wählt die Fußsteuerung jenes Gerät zur drahtlosen Übertragung von Betriebsdaten, insbesondere Steuerungsdaten für die Behandlung und/ oder Diagnose aus, von dem die Fußsteuerung ein Datenpaket empfangen hat und dessen individueller Rang einen im Vergleich zu den übrigen Geräten, von denen die Fußsteuerung ein Datenpaket empfangen hat, priorisierten Rang einnimmt oder Wert aufweist. Der priorisierte, individuelle Rang kann im Vergleich zu den anderen individuellen Rängen der aktiven Geräte zum Beispiel eine bevorzugte oder übergeordnete oder höhere oder niedrigere Position in der Rangordnung oder in einer Rangliste oder einen bevorzugten oder übergeordneten oder höheren oder niedrigeren Wert aufweisen.

Besonders bevorzugt speichert oder zwischenspeichert die Fußsteuerung die empfangenen Datenpakete der zwei oder mehr durch die Fußsteuerung ansteuerbaren Geräten, insbesondere in einem Speicherelement der Fußsteuerung. Die Steuerlogik der Fußsteuerung greift auf diese empfangenen und gespeicherten Datenpakete zu und vergleicht insbesondere die individuellen Ränge der empfangenen und gespeicherten Datenpakete. Basierend auf diesem Vergleich wählt die Steuerlogik den Identifikationscode, d.h. das Gerät mit dem priorisierten Rang aus, zu dem die Fußsteuerung eine drahtlose Kommunikation zur Übertragung von Betriebsdaten aufbaut.

Gemäß einer Ausführungsform der Erfindung umfasst das medizinische oder dentale System exakt zwei Geräte, die mit der Fußsteuerung steuerbar sind. Diesen zwei Geräten ist jeweils ein Datenpaket mit einem spezifischen Identifikationscode und einem individuellen Rang zugeordnet, die insbesondere in einem Speicherelement des jeweiligen Geräts gespeichert sind. Ein Datenpaket eines Geräts umfasst zum Beispiel den Identifikationscode WHTW und den Rang 10, das Datenpaket des anderen Geräts umfasst zum Beispiel den Identifikationscode WHUL und den Rang 5. Sind beide Geräte aktiv und senden beide Geräte (an einem bestimmten Zeitpunkt oder während eines begrenzten Zeitraums) gleichzeitig ihr jeweiliges Datenpaket und empfängt die (gemeinsame) Fußsteuerung beide Datenpakete, so vergleicht die Steuerlogik die in den jeweiligen Datenpaketen enthaltenen, individuellen Ränge der zwei Geräte. Ist die Steuerlogik zum Beispiel derart ausgebildet, dass der höhere individuelle Rang oder Wert der Rangordnung priorisiert ist, so baut die Fußsteuerung eine drahtlose Kommunikationsverbindung zur Übertragung von Betriebsdaten oder Steuerungsdaten für die Behandlung und/ oder Diagnose nur zu dem Gerät mit dem Identifikationscode WHTW (mit dem individuellen Rang 10) auf. Entsprechend diesem Ausführungsbeispiel baut die Fußsteuerung somit keine drahtlose Kommunikationsverbindung zur Übertragung von Betriebsdaten zu dem Gerät mit dem Identifikationscode WHUL (mit dem individuellen Rang 5) auf.

Das im Vorstehenden beschriebene Ausführungsbeispiel gilt entsprechend für medizinische oder dentale System mit mehr als zwei Geräten, die mit der Fußsteuerung steuerbar sind, und bei denen (an einem bestimmten Zeitpunkt oder während eines begrenzten Zeitraums) mehr als zwei Geräte gleichzeitig ihr jeweiliges Datenpaket senden. Wiederum vergleicht die Steuerlogik die individuellen Ränge der empfangenen Datenpakete. Basierend auf diesem Vergleich baut die Fußsteuerung eine drahtlose Kommunikationsverbindung zur Übertragung von Betriebsdaten oder Steuerungsdaten für die Behandlung und/ oder Diagnose nur zu jenem Gerät auf, dessen individueller Rang priorisiert ist.

Vorzugsweise ist vorgesehen, dass, wenn die Fußsteuerung (an einem bestimmten Zeitpunkt oder während eines begrenzten Zeitraums) nur von einem einzigen durch die Fußsteuerung ansteuerbaren Gerät einen spezifischen Identifikationscode oder ein Datenpaket empfängt, dann wählt die Steuerlogik der Fußsteuerung dieses Gerät zur drahtlosen Übertragung von Betriebsdaten oder Steuerungsdaten für die Behandlung und/ oder Diagnose aus. Damit ist in vorteilhafter Weise sichergestellt, dass das Verfahren auch durchführbar ist, wenn nur eines der mehreren durch die Fußsteuerung ansteuerbaren Geräte aktiv oder eingeschalten ist. Insbesondere ist die Steuerlogik ausgebildet, bei Empfang nur eines spezifischen Identifikationscodes oder Datenpakets keine Ermittlung oder keinen Vergleich der individuellen Ränge durchzuführen.

Vorzugsweise ist bei dem Verfahren zur drahtlosen Übertragung von Daten vorgesehen, dass Datenpakete, insbesondere die individuellen Ränge der durch die Fußsteuerung ansteuerbaren Geräte für den Anwender unveränderbar sind. Der Anwender kann somit insbesondere den Rang eines Geräts, zum Beispiel dessen Wert oder Position in einer Rangordnung oder Rangliste, nicht verändern. Beispielsweise werden die individuellen Ränge der Geräte während der Herstellung der Geräte oder der Fußsteuerung bestimmt und gespeichert. Damit wird in vorteilhafter Weise eine Fehlbedienung durch den Anwender verhindert.

Alternativ ist bei dem Verfahren zur drahtlosen Übertragung von Daten vorgesehen, dass die individuellen Ränge der durch die Fußsteuerung ansteuerbaren Geräte für den Anwender veränderbar sind. Der Anwender kann somit insbesondere den Rang eines Geräts, zum Beispiel dessen Wert in einem Datenpaket oder dessen Wert oder Position einer Rangordnung oder Rangliste, verändern oder auswählen. Damit wird dem Anwender in vorteilhafter Weise eine größere individuelle Freiheit bei dem Verfahren und bei der Bedienung des medizinischen oder dentalen Systems ermöglicht.

Vorzugsweise ist vorgesehen, dass die individuellen Ränge der Datenpakete und/ oder der Rangordnung durch das Pairen und/ oder während des Pairens (d.h. das erstmalige kommunikative in Verbindung Bringen) der durch die Fußsteuerung ansteuerbaren Geräte mit der Fußsteuerung bestimmt werden. Da das medizinische oder dentale System mehrere Geräte aufweist, die wahlweise und drahtlos mit einer gemeinsamen Fußsteuerung kommunizieren und durch diese ansteuerbar sind, ist es notwendig vor der erstmaligen Ansteuerung der Geräte durch die Fußsteuerung jedes der Geräte mit der Fußsteuerung zu pairen. Durch das Festlegen der individuellen Ränge durch das Pairen und/ oder während das Pairens ist in vorteilhafter Weise eine für den Anwender sehr einfache Möglichkeit geschaffen, die individuellen Ränge oder Rangordnung der Geräte zu bestimmen, insbesondere nach seinen eigenen Wünschen festzulegen. Insbesondere wird für das Bestimmen der individuellen Ränge in vorteilhafter Weise auch keine zusätzliche Zeit benötigt.

Vorzugsweise ist vorgesehen, dass die individuellen Ränge durch die Reihenfolge, in welcher die durch die Fußsteuerung ansteuerbaren Geräte mit der Fußsteuerung gepairt werden, bestimmt werden. Beispielsweise erhält jenes Gerät, das als erstes mit der Fußsteuerung gepairt wird den höchsten oder niedrigsten Rang oder Wert oder den Rang oder Wert 1, das Gerät, das als nächstes mit der Fußsteuerung gepairt wird, den nächst hohen oder niedrigen Rang oder Wert oder den Rang oder Wert 2, das darauffolgend mit der Fußsteuerung gepairte Gerät den nächst hohen oder niedrigen Rang oder Wert oder den Rang oder Wert 3, usw.

Alternativ oder zusätzlich ist vorgesehen, dass die individuellen Ränge durch für das Pairen verwendete Treiber und/ oder die Art des Pairings zwischen der Fußsteuerung und der durch die Fußsteuerung ansteuerbaren Geräte bestimmt werden. Beispielsweise ist ein Treiber zum drahtgebundenen Pairen und ein Treiber zum drahtlosen Pairen vorgesehen. Die Art des Pairings umfasst zum Beispiel eine drahtgebundene Verbindung und eine drahtlose Verbindung. Beispielsweise erhält jenes Gerät, das drahtlos mit der Fußsteuerung gepairt wird oder bei dem ein Treiber zum drahtlosen Pairen verwendet wird, einen Rang oder Wert 1 und ein Gerät, das drahtgebunden mit der Fußsteuerung gepairt wird oder bei dem ein Treiber zum drahtgebunden Pairen verwendet wird, einen Rang oder Wert 2.

Die beiden im Vorstehenden beschriebenen Möglichkeiten zur Ermittlung der individuellen Ränge werden durch das Pairing und während des Pairings bewirkt. Alternativ oder zusätzlich kann vorgesehen sein, dass die individuellen Ränge während des Pairings zwischen der Fußsteuerung und der durch die Fußsteuerung ansteuerbaren Geräte durch eine Auswahl des Anwenders des medizinischen oder dentalen Systems festgelegt werden. Beispielsweise wählt der Anwender über ein Stellelement oder ein Bedienfeld an der Fußsteuerung oder an einem Gerät den individuellen Rang des Geräts, das gerade mit der Fußsteuerung gepairt wird, aus.

Die durch das Pairing und/ oder während des Pairings festgelegten individuellen Ränge werden vorzugsweise während oder nach Abschluss des Pairings in dem Speicherelement des Geräts und/ oder der Fußsteuerung gespeichert, insbesondere gemeinsam mit oder ergänzend zu dem Identifikationscode des jeweiligen Geräts, um so insbesondere die Datenpakete zu bilden.

Vorzugsweise ist bei dem Verfahren zur drahtlosen Übertragung von Daten vorgesehen, dass die Fußsteuerung durch den Empfang eines Identifikationscodes oder Datenpakets eines Geräts aus einem Modus mit zumindest verringerter Sendeleistung in einen Betriebsmodus mit im Vergleich zur zumindest verringerten Sendeleistung erhöhter Sendeleistung versetzt wird. Damit wird die Fußsteuerung in vorteilhafter Weise in den Betriebsmodus versetzt, ohne dass der Anwender aktiv die Fußsteuerung betätigen muss. Alternativ betätigt der Anwender eine Benutzerschnittstelle oder ein Stellelement der Fußsteuerung, um diese in den Betriebsmodus zu versetzen.

Vorzugsweise ist bei dem Verfahren zur drahtlosen Übertragung von Daten vorgesehen, dass das Gerät nach der Auswahl durch die Fußsteuerung zur Übertragung der Betriebsdaten, insbesondere der Steuerungsdaten und/ oder während der Übertragung der Betriebsdaten zumindest seinen spezifischen Identifikationscode oder sein Datenpaket an die Fußsteuerung sendet, insbesondere wiederholt und/ oder innerhalb vorbestimmter Zeitintervalle, um die drahtlose Kommunikation zwischen der Fußsteuerung und dem Gerät aufrecht zu erhalten.

Vorzugsweise ist bei dem Verfahren zur drahtlosen Übertragung von Daten vorgesehen, dass vor der Auswahl eines Geräts durch die Steuerlogik der Fußsteuerung das Senden eines spezifischen Identifikationscodes oder eines Datenpakets von einem Gerät durch den Anwender bewirkt wird, zum Beispiel durch Betätigen eines Stellelements des Geräts oder durch das Bewegen oder in die Hand nehmen des Geräts. Das Bewegen oder in die Hand nehmen des Geräts wird beispielsweise durch einen Lagesensor oder einen Drucksensor des Geräts erkannt. Das Stellelement oder die Sensoren erzeugen vorzugsweise ein Stellsignal, das von einer Steuereinheit empfangen wird, die als Reaktion darauf ausgebildet ist, den spezifischen Identifikationscode und gegebenenfalls den individuellen Rang des Geräts zu senden. Damit ist in vorteilhafter Weise sichergestellt, dass automatisch, sobald ein Anwender ein vorher nicht benutztes Gerät in die Hand nimmt, ein Identifikationscode an die Fußsteuerung gesendet wird.

Vorzugsweise umfasst das Verfahren zur drahtlosen Übertragung von Daten in einem medizinischen oder dentalen System des Weiteren, dass eine aufgebaute oder bestehende Kommunikationsverbindung zwischen der Fußsteuerung und einem der Geräte unterbrochen oder beendet wird. Die Kommunikationsverbindung zwischen der Fußsteuerung und einem der Geräte ist somit reversibel. Vorzugsweise wird die Kommunikationsverbindung zeitabhängig unterbrochen oder beendet, zum Beispiel nach Ablauf einer, insbesondere vorbestimmten, Zeitdauer, vorzugsweise nach Ablauf von einer Sekunde oder von mehreren Sekunden, zum Beispiel von fünf oder zehn Sekunden. Besonders bevorzugt wird die Kommunikationsverbindung zeitabhängig unterbrochen oder beendet, wenn die genannte Zeitdauer nach der letzten Abgabe von drahtlosen Betriebsdaten oder Steuerungsdaten für die Behandlung und/ oder Diagnose an das Gerät, das in Kommunikationsverbindung mit der Fußsteuerung steht, verstrichen ist. Damit wird in vorteilhafter Weise sichergestellt, dass eine aktuell vorhandene, aufgebaute Kommunikationsverbindung zwischen der Fußsteuerung und einem ersten Gerät nicht den Aufbau einer neuen Kommunikationsverbindung, insbesondere zwischen der Fußsteuerung und einem anderen, zweiten Gerät behindert.

Vorzugsweise ist die Fußsteuerung ausgebildet, nach Ablauf der genannten Zeitdauer oder nach Unterbrechung der Kommunikationsverbindung in einen Modus mit zumindest verringerter Sendeleistung (einen Standby-Modus oder Sleep-Modus) einzutreten, der im Vergleich zu einem Betriebsmodus während der Kommunikation mit dem Gerät eine zumindest verringerte Sendeleistung aufweist.

Vorzugsweise sind die durch die Fußsteuerung ansteuerbaren Geräte ausgebildet, nach Ablauf der genannten Zeitdauer oder nach Unterbrechung der Kommunikationsverbindung in einen Modus mit zumindest verringerter Sendeleistung (einen Standby-Modus oder Sleep-Modus) einzutreten, der im Vergleich zu einem Betriebsmodus während der Kommunikation mit der Fußsteuerung eine zumindest verringerte Sendeleistung aufweist. Vorzugsweise senden die Geräte in dem Modus mit zumindest verringerter Sendeleistung keinen Identifikationscode oder kein Datenpaket.

Nach der Unterbrechung der Kommunikationsverbindung kann diese zwischen dem gleichen oder einem anderen Gerät und der Fußsteuerung auf Basis der individuellen Ränge und der in der Fußsteuerung vorgesehenen Steuerlogik erneut aufgebaut werden, so wie dies im Vorstehenden beschrieben ist.

Vorzugsweise werden die von den Geräten gesendeten, der Fußsteuerung empfangenen und in dem Speicherelement der Fußsteuerung gespeicherten, spezifischen Identifikationscodes und/ oder Datenpakete nach dem Aufbau einer drahtlosen Kommunikationsverbindung zur Übertragung von Betriebsdaten an ein Gerät oder mit Beendigung der Übertragung von Betriebsdaten aus dem Speicherelement der Fußsteuerung gelöscht. Damit wird in vorteilhafter Weise ein neuer Verbindungsaufbau zu einem der Geräte ermöglicht.

Ein Computerprogrammprodukt oder computerlesbares Speichermedium umfasst Befehle, die bei der Ausführung durch einen Computer diesen veranlassen, das im Vorstehenden beschriebene Verfahren zur drahtlosen Übertragung von Daten in einem medizinischen oder dentalen System, das eine Fußsteuerung und mehrere medizinische oder dentale Geräte zur Behandlung und/ oder Diagnose umfasst, auszuführen. Das Computerprogrammprodukt, das computerlesbare Speichermedium und der Computer sind insbesondere Teile des medizinischen oder dentalen Systems, zum Beispiel der Fußsteuerung und/ oder der Steuerlogik, oder sind zumindest operativ mit diesem verbunden.

Eine Fußsteuerung zur wahlweisen drahtlosen Steuerung eines von zumindest zwei durch die Fußsteuerung ansteuerbaren, medizinischen oder dentalen Geräten, umfasst: zumindest ein Stellelement zum Stellen eines Wertes von Betriebsdaten oder Steuerungsdaten für die Behandlung und/ oder Diagnose für die zumindest zwei Geräte, eine Sende- und Empfangseinheit zur bidirektionalen, drahtlosen Kommunikation mit den zumindest zwei Geräten einschließlich der Übertragung der durch das Stellelement stellbaren Betriebsdaten, und eine Vorrichtung zur Datenverarbeitung mit Mitteln zur Ausführung des im Vorstehenden beschriebenen Verfahrens. Vorzugsweise umfasst die Vorrichtung zur Datenverarbeitung die Steuerlogik zur Auswahl jenes Geräts, an welches die Fußsteuerung drahtlos Betriebsdaten oder Steuerungsdaten für die Behandlung und/ oder Diagnose überträgt.

Die Fußsteuerung oder Funk-Fußsteuerung des medizinischen oder dentalen Systems umfasst insbesondere zumindest eines der folgenden Elemente:
- eine Sende- und Empfangseinheit oder einen Transceiver zum Senden und Empfangen von Daten und/ oder elektromagnetischer Energie, insbesondere zur Kommunikation mit einer Sende- und Empfangseinheit der durch die Fußsteuerung ansteuerbaren Geräte. Die Sende- und Empfangseinheit ist insbesondere zur bidirektionalen, drahtlosen Kommunikation mit den zumindest zwei durch die Fußsteuerung ansteuerbaren Geräten des medizinischen oder dentalen Systems ausgebildet. Die Sende- und Empfangseinheit ist zum Beispiel ausgebildet, Identifikationscodes und/ oder Datenpakete der Geräte zu empfangen und Betriebsdaten oder Steuerungsdaten für die Behandlung und/ oder Diagnose an die zumindest zwei Geräte zu senden. Die Sende- und Empfangseinheit umfasst zum Beispiel einen Funksender und/ oder eine Antenne und/ oder elektrische Leiter, die mit der Steuerlogik verbunden sind und die zur Übertragung von Daten und/ oder elektromagnetischer Energie zwischen der Sende- und Empfangseinheit und der Steuerlogik ausgebildet sind;
- eine Energiequelle zur Energieversorgung der Fußsteuerung, insbesondere der Sende- und Empfangseinheit und gegebenenfalls weiterer elektrische Energie benötigender Komponenten der Fußsteuerung, insbesondere eines Mikrocontrollers. Die Energiequelle umfasst insbesondere einen wiederholt aufladbaren Akkumulator;
- zumindest eine Benutzerschnittstelle, insbesondere ein dreh- oder schwenkbares Pedal oder Stellelement, über die der Anwender Stellbefehle, zum Beispiel Betriebsdaten oder Steuerungsdaten für die Behandlung und/ oder Diagnose, erzeugt;
- einen operativ mit der Benutzerschnittstelle und der Sende- und Empfangseinheit verbundenen Mikrocontroller. Vorzugsweise umfasst der Mikrocontroller die Steuerlogik, welche zum Verarbeiten oder Vergleichen der Identifikationscodes oder der Datenpakete der Geräte und zur Auswahl jenes Geräts ausgebildet ist, an welches drahtlos Betriebsdaten oder Steuerungsdaten für die Behandlung und/ oder Diagnose übertragen werden. Vorzugsweise umfasst die im Vorstehenden beschriebene Vorrichtung zur Datenverarbeitung den Mikrocontroller. Alternativ oder zusätzlich ist der Mikrocontroller zum Beispiel ausgebildet ist, die über die Benutzerschnittstelle erzeugten Stellbefehle des Anwenders zu empfangen, vorzugsweise zu verarbeiten und an die Sende- und Empfangseinheit weiterzuleiten, so dass die Sende- und Empfangseinheit Stellbefehle, zum Beispiel Betriebsdaten oder Steuerungsdaten, an die Geräte für die Behandlung und/ oder Diagnose überträgt;
- ein Speicherelement, in dem insbesondere die den jeweiligen Geräten zugeordneten, spezifischen Identifikationscodes und/ oder die Datenpakete gespeichert oder speicherbar ist. Das Speicherelement ist vorzugsweise als Teil des Mikrocontrollers ausgebildet oder mit dem Mikrocontroller operativ verbunden, so dass insbesondere Daten, zum Beispiel Identifikationscodes oder Datenpakete, zwischen dem Speicherelement und dem Mikrocontroller übertragbar sind.

Die Fußsteuerung ist als gemeinsame Fußsteuerung für die zumindest zwei durch die Fußsteuerung ansteuerbaren Geräte ausgebildet. Vorzugsweise kommuniziert jedes der durch die Fußsteuerung ansteuerbaren Geräte direkt mit der Fußsteuerung und umgekehrt.

Vorzugsweise umfasst zumindest eines der durch die Fußsteuerung ansteuerbaren Geräte oder Handstücke:
- einen Griffabschnitt zum Halten des Geräts;
- einen Kopfabschnitt, der zum Beispiel umfasst: eine Werkzeughalterung zum lösbaren Halten eines Behandlungswerkzeugs und/ oder eine Abgabevorrichtung zur Abgabe eines Behandlungsmediums und/ oder einen Sensor zur Messung oder Bestimmung eines Betriebsparameters oder eines Wertes oder Zustands eines Körpergewebes;
- eine Sende- und Empfangseinheit oder einen Transceiver zum Senden und Empfangen von Daten und/ oder elektromagnetischer Energie, insbesondere zur Kommunikation mit der Sende- und Empfangseinheit der Fußsteuerung. Die Sende- und Empfangseinheit ist insbesondere zur bidirektionalen, drahtlosen Kommunikation mit der Fußsteuerung des medizinischen oder dentalen Systems ausgebildet. Die Sende- und Empfangseinheit ist zum Beispiel ausgebildet, den spezifischen Identifikationscode oder das Datenpaket des Geräts an die Fußsteuerung zu senden und Betriebsdaten oder Steuerungsdaten für die Behandlung und/ oder Diagnose von der Fußsteuerung zu empfangen. Die Sende- und Empfangseinheit umfasst zum Beispiel einen Funksender, eine Antenne und/ oder elektrische Leiter, die mit einer Steuereinheit des Geräts verbunden sind. Die Sende- und Empfangseinheit kann entweder im Handstück selbst oder in einer operativ, insbesondere über eine Leitung und/ oder ein Kabel, damit verbundenen Versorgungs- und/ oder Steuereinheit vorgesehen sein;
- eine Energiequelle zur Energieversorgung des Geräts, insbesondere der Sende- und Empfangseinheit und gegebenenfalls weiterer elektrische Energie benötigender Komponenten der Geräte, insbesondere eines Mikrocontrollers. Die Energiequelle umfasst insbesondere einen wiederholt aufladbaren Akkumulator;
- ein Stell- oder Schaltelement, um bei dessen Betätigung durch den Anwender zum Beispiel ein Modus mit zumindest verringerter Sendeleistung (Standby-Modus oder Sleep-Modus) des Geräts zu beenden und das Gerät in einen Betriebsmodus mit im Vergleich erhöhter Sendeleistung zu versetzen, in dem das Gerät aktiv ist und/ oder zum Senden des Identifikationscodes oder des Datenpakets bereit ist oder sendet und/ oder zum Empfangen von Daten der Fußsteuerung, insbesondere der Betriebsdaten oder Steuerungsdaten für die Behandlung und/ oder Diagnose, bereit ist oder diese empfängt und/ oder zum Betrieb bereit ist. Das Stell- oder Schaltelement umfasst zum Beispiel einen Taster, einen Drucksensor oder einen Lagesensor.
- eine Steuereinheit zur Steuerung des Geräts, insbesondere von Behandlungs- und/ oder Diagnosemodi oder von Werten von Betriebsparametern, zum Beispiel der Drehzahl, und/ oder zum Empfangen von Sensordaten eines im Vorstehenden genannten Sensors des Geräts. Besonders bevorzugt ist die Steuereinheit mit dem Stell- oder Schaltelement und/ oder mit der Sende- und Empfangseinheit verbunden, so dass die drahtlose Übertragung des Identifikationscodes oder des Datenpakets an die Fußsteuerung durch die Steuereinheit steuerbar ist und/ oder dass von der Sende- und Empfangseinheit empfangene Betriebsdaten oder Steuerungsdaten für die Behandlung und/ oder Diagnose an die Steuereinheit zur Steuerung des Geräts weiterleitbar sind;
- ein Speicherelement, in dem der dem Gerät zugeordnete, spezifische Identifikationscode oder das das jeweilige Datenpaket gespeichert oder speicherbar sind. Das Speicherelement ist vorzugsweise als Teil der Steuereinheit ausgebildet oder mit der Steuereinheit operativ verbunden, so dass insbesondere Daten, zum Beispiel der Identifikationscode oder das Datenpaket, zwischen dem Speicherelement, dem Mikrocontroller und der Sende- und Empfangseinheit übertragbar sind.

Vorzugsweise umfasst zumindest eines der durch die Fußsteuerung ansteuerbaren Geräte ein medizinisches oder dentales Prophylaxe-Gerät, zum Beispiel ein Handstück oder Gerät zur Entfernung von Zahnstein oder Plaque oder Verfärbungen oder ein Handstück oder Gerät zur Abgabe eines Behandlungsmediums, insbesondere eines Pulvers auf eine Behandlungsstelle. Alternativ oder zusätzlich umfasst zumindest eines der durch die Fußsteuerung ansteuerbaren Geräte ein Handstück oder Gerät zur Bearbeitung von Knochengewebe oder Knochenersatzmaterial, zum Beispiel eine Säge oder einen rotierenden oder oszillierenden Bohrer, oder ein Handstück oder Gerät zur Behandlung und/ oder Vermessung eines Körperhöhle oder Kavität, zum Beispiel eines Wurzelkanals oder der Nasehöhle oder des Gehörganges und/ oder ein Handstück oder Gerät zur Diagnose, zum Beispiel von Karies oder krankhaften Gewebsveränderungen.

Vorzugsweise umfasst zumindest eines der durch die Fußsteuerung ansteuerbaren Geräte ein medizinisches oder dentales Handstück oder ist durch ein (drahtloses) Handstück gebildet. Vorzugsweise ist das Handstück Teil eines Tischgeräts und operativ über eine Leitung mit einer Versorgungs- und/ oder Steuereinheit verbunden.

Ein medizinisches oder dentales System umfasst eine im Vorstehenden beschriebene Fußsteuerung und mehrere medizinische oder dentale Geräte zur Behandlung und/ oder Diagnose, wobei zumindest zwei der mehreren Geräte durch die Fußsteuerung ansteuerbar sind und die Fußsteuerung ausgebildet ist, wahlweise (und reversibel) eines der zumindest zwei Geräte durch die Fußsteuerung anzusteuern. Die mehreren medizinischen oder dentalen Geräte umfassen insbesondere zumindest eines der im Vorstehenden beschriebenen Geräte. Das medizinische oder dentale System ist insbesondere als Tischgerät-System ausgebildet.

Die Erfindung wird nachfolgend anhand bevorzugter Ausführungsbeispiele und Bezug nehmend auf die beigefügten Zeichnungen erläutert:
Figur 1 zeigt schematisch ein medizinisches oder dentales System, in dem wahlweise drahtlos Daten und/ oder elektromagnetische Energie zwischen einer Fußsteuerung und einem von mehreren medizinischen oder dentalen Geräten übertragen werden.
Figur 2 zeigt schematisch die Speicherelemente der medizinischen oder dentalen Geräte der Figur 1 mit einem jeweils darin gespeicherten, spezifischen Identifikationscode.
Figur 3 zeigt schematisch das Speicherelement der Fußsteuerung der Figur 1 mit mehreren, darin gespeicherten Datenpaketen.
Figur 4 zeigt schematisch ein alternatives medizinisches oder dentales System, in dem wahlweise drahtlos Daten und/ oder elektromagnetische Energie zwischen einer Fußsteuerung und einem von mehreren medizinischen oder dentalen Geräten übertragen werden.
Figur 5 zeigt schematisch die Speicherelemente der medizinischen oder dentalen Geräte der Figur 4 mit einem jeweils darin gespeicherten, spezifischen Datenpaket.
Figur 6 zeigt ein weiteres medizinisches oder dentales System, in dem wahlweise drahtlos Daten und/ oder elektromagnetische Energie zwischen einer Fußsteuerung und einem von mehreren medizinischen oder dentalen Geräten übertragen werden
Figur 7 die Fußsteuerung und eines der mehreren medizinischen oder dentalen Geräte der Figur 6 während des Pairings.

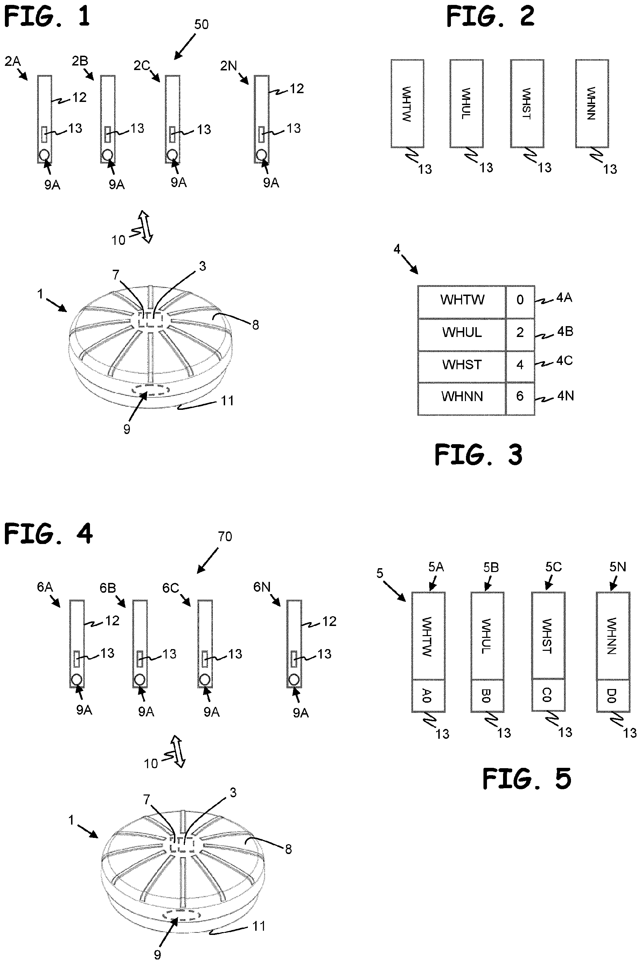

Die in den Figuren 1, 4 und 6 dargestellten medizinischen oder dentalen Systeme 50, 70, 100 umfassen jeweils eine Fußsteuerung 1 und mehrere medizinische oder dentale Geräte 2, 2A-2N; 6, 6A-6N zur Behandlung und/ oder Diagnose. Die Geräte 2, 2A-2N; 6, 6A-6N und die Fußsteuerung 1 sind derart ausgebildet, dass zwischen der Fußsteuerung 1 und den Geräten 2, 2A-2N; 6, 6A-6N Daten und/ oder elektromagnetische Energie drahtlos mittels Funk übertragbar ist (dies ist durch den Pfeil 10 symbolisiert). Jedes der Geräte 2, 2A-2N; 6, 6A-6N ist wahlweise durch die Fußsteuerung 1 drahtlos ansteuerbar, zum Beispiel mit Steuerungsdaten zum Betrieb des jeweiligen Geräts 2, 2A-2N; 6, 6A-6N, insbesondere mit Steuerungsdaten für die Behandlung und/ oder Diagnose. Zur bidirektionalen Kommunikation und Übertragung von Daten und/ oder elektromagnetischer Energie weisen die Fußsteuerung 1 und jedes der Geräte 2, 2A-2N; 6, 6A-6N eine Sende- und Empfangseinheit 9, 9A zum Senden und Empfangen von Daten und/ oder elektromagnetischer Energie auf.

Die Fußsteuerung 1 umfasst unter anderem eine Bodenplatte 11, zumindest ein relativ zur Bodenplatte 11 insbesondere dreh- oder schwenkbares Stellelement 8, über das der Anwender Stellbefehle, zum Beispiel Betriebsdaten oder Steuerungsdaten für die Behandlung und/ oder Diagnose, erzeugt und eine Steuerung oder einen Mikrocontroller oder einen Computer 7. Der Mikrocontroller 7 bildet die zentrale Steuereinheit der Fußsteuerung 1 und ist mit dem Stellelement 8 und der Sende- und Empfangseinheit 9 verbunden, um zum Beispiel Stellbefehle des Stellelements 8 und/ oder Steuerungsdaten für die Behandlung und/ oder Diagnose und/ oder von den Geräten 2, 2A-2N; 6, 6A-6N erhaltene Daten, zum Beispiel einen spezifischen Identifikationscode oder ein Datenpaket eines der Geräten 2A-2N; 6A-6N zu empfangen und/ oder zu verarbeiten und / oder weiterzuleiten.

Die Fußsteuerung 1 umfasst des Weiteren eine Steuerlogik 3, die ausgebildet ist, auf Basis von spezifischen, jeweils einem der Geräte 2, 2A-2N; 6, 6A-6N zugeordneten Datenpaketen 4A-4N; 5A-5N, eines der durch die Fußsteuerung 1 ansteuerbaren Geräte 2, 2A-2N; 6, 6A-6N auszuwählen, an das drahtlos Betriebsdaten oder Steuerungsdaten für die Behandlung und/ oder Diagnose übertragen werden. Die Steuerlogik 3 ist insbesondere als Teil des Mikrocontrollers oder Computers 7 ausgebildet.

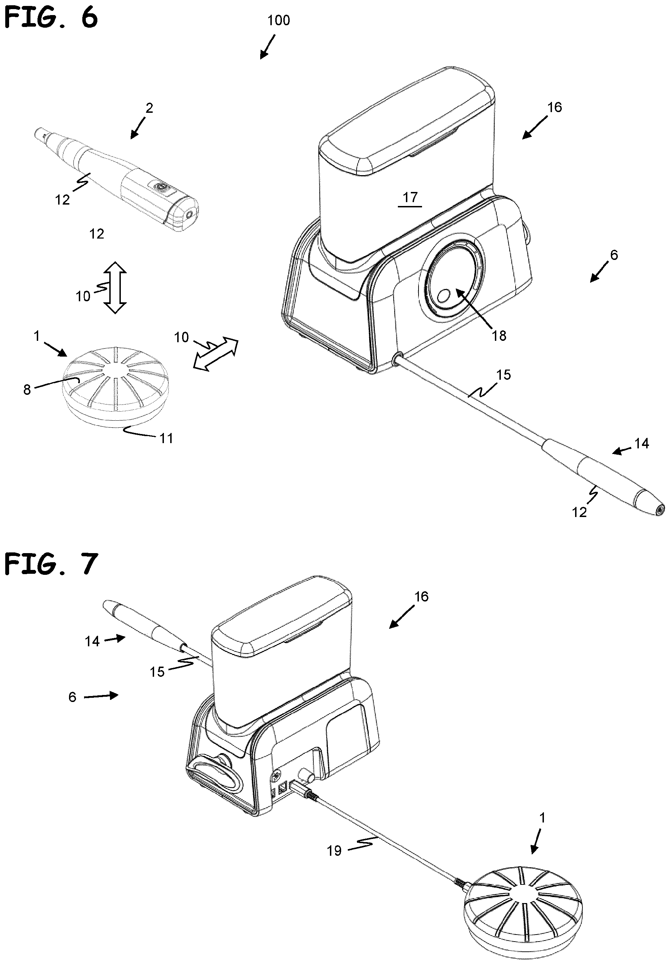

Die in den Figuren 1 und 4 nur schematisch dargestellten Geräte 2A-2N; 6A-6N sowie die Geräte 2, 6 der Figuren 6, 7 weisen eine Außenhülse 12 auf, die insbesondere auch zum Halten der Geräte 2, 2A-2N; 6, 6A-6N mit einer Hand dienen. Jedes Gerät 2, 2A-2N; 6, 6A-6N umfasst eine Sende- und Empfangseinheit 9A zur drahtlosen Kommunikation 10 mit der Fußsteuerung 1, insbesondere zum drahtlosen Übertragen von Daten und/ oder elektromagnetischer Energie zwischen dem Gerät 2, 2A-2N; 6, 6A-6N und der Fußsteuerung 1, und ein Speicherelement 13. Das Speicherelement 13 und die Sende- und Empfangseinheit 9A jedes Geräts 2, 2A-2N; 6, 6A-6N sind kommunikativ miteinander verbunden, so dass in dem Speicherelement 13 gespeicherte Daten an die Sende- und Empfangseinheit 9A übertragen und von dieser gesendet werden und optional Daten von der Sende- und Empfangseinheit 9A empfangen und an das Speicherelement 13 zur Speicherung weitergeleitet werden.

Im Inneren oder an der Außenhülse 12 können je nach Art und Verwendungszweck der Geräte 2, 2A-2N; 6, 6A-6N unterschiedliche Bauteile vorgesehen sein, wie zum Beispiel Kupplungselemente, Medienleitungen, Medienabgabevorrichtungen oder- öffnungen, Strahlungsquellen, Strahlungsleiter, Sensoren, elektrische Leitungen, Werkzeughaltevorrichtungen, Antriebselemente, Stellelemente, Anzeigevorrichtungen, eine Steuereinheit zur Steuerung des Geräts usw.

Im Folgenden wird das Ausführungsbeispiel gemäß den Figuren 1 - 3 weiter erläutert. Die Figur 2 zeigt schematisch die Speicherelemente 13 der Geräte 2A-2N (in den Geräten 2A-2N entsprechender Reihung), wobei in jedem der Speicherelemente 13 ein spezifischer Identifikationscode gespeichert ist. Der Identifikationscode ist beispielhaft für das erste Gerät 2A mit "WHTW", für das zweite Geräte 2B mit "WHUL", für das dritte Gerät 2C mit "WHST" und für das vierte Gerät 2N als "WHNN" angegeben. Jeder spezifische Identifikationscode ist einem bestimmten Gerät 2A-2N zugeordnet, nämlich jenem Gerät 2A-2N, in dessen Speicherelement 13 er gespeichert ist. Jeder spezifische Identifikationscode ist eindeutig und von jedem anderen Identifikationscode unterscheidbar. Somit kann jedes Gerät 2A-2N auf Basis des oder seines spezifischen Identifikationscodes eindeutig identifiziert werden.

Die Figur 3 zeigt vier Datenpakete 4, 4A-4N, die gemäß dem Ausführungsbeispiel der Figuren 1 - 3 in dem Stellelement 8, insbesondere in einem Speicherelement des Mikrocontrollers oder Computers 7, gespeichert sind. Jedes der Datenpakete 4A-4N umfasst einen der spezifischen Identifikationscodes (WHTW, WHUL, WHST, WHNN) der durch die Fußsteuerung 1 ansteuerbaren Geräte 2A-2N und einen jedem Gerät 2A-2N zugeordneten, individuellen, eindeutigen Rang einer Rangordnung der durch die Fußsteuerung 1 ansteuerbaren Geräte 2A-2N. Die Ränge oder die Werte der Ränge sind beispielhaft mit "0", "2", "4" und "6" bezeichnet, wobei dem Gerät 2A mit dem Identifikationscode "WHTW" der Rang "0", dem Gerät 2B mit dem Identifikationscode "WHUL" der Rang "2", dem Gerät 2C mit dem Identifikationscode "WHST" der Rang "4" und dem Gerät 2N mit dem Identifikationscode "WHNN" der Rang "6" zugeordnet ist. Somit ist (über den Identifikationscode) jedem der durch die Fußsteuerung 1 ansteuerbaren Gerät 2A-2N ein eigenes, spezifisches Datenpaket 4 und dadurch ein individueller, eindeutiger Rang zugeordnet.

Die Auswahl an welches der durch die Fußsteuerung 1 ansteuerbaren Gerät 2A-2N drahtlos Betriebsdaten oder Steuerungsdaten übertragen werden geschieht gemäß dem Ausführungsbeispiel der Figuren 1-3 wie folgt: damit Betriebsdaten an ein Gerät 2A-2N übertragen werden können, muss das Gerät 2A-2N aktiv sein oder sich in einem Betriebsmodus befinden, in dem es insbesondere zum drahtlosen Senden und Empfangen von Daten bereit ist. Das Aktivieren des Geräts 2A-2N oder das in den Betriebsmodus Versetzen kann beispielsweise durch den Anwender bewirkt werden, in dem dieser das Gerät 2A-2N ergreift, bewegt und/ oder ein Stellelement betätigt. Im Folgenden sendet ein aktives Gerät 2A-2N seinen jeweiligen, spezifischen Identifikationscode drahtlos über die Sende- und Empfangseinheit 9A an die Fußsteuerung 1.

Sind mehrere Geräte 2A-2N aktiv oder senden (innerhalb eines vorbestimmten Zeitraums) mehrere Geräte 2A-2N ihren jeweils spezifischen Identifikationscode, so empfängt die Fußsteuerung 1 diese spezifischen Identifikationscodes und leitet diese an die Steuerlogik 3 weiter. Die Steuerlogik 3 erkennt aufgrund der empfangenen Identifikationscodes welche Geräte 2A-2N aktiv sind. Durch Vergleich der in den Datenpaketen 4 hinterlegten Identifikationscodes der Geräte 2A-2N mit den empfangenen Identifikationscodes der aktiven Geräte 2A-2N ermittelt die Steuerlogik des Weiteren welchen individuellen Rang die aktiven Geräte 2A-2N einnehmen. In Abhängigkeit davon welcher individuelle Rang der aktiven Geräte 2A-2N priorisiert ist, wählt die Steuerlogik oder Fußsteuerung 1 jenes Gerät 2A-2N zur ausschließlichen drahtlosen Übertragung von Betriebsdaten, insbesondere Steuerungsdaten für die Behandlung und/ oder Diagnose, aus, von dem die Fußsteuerung 1 einen Identifikationscode empfangen hat und das in der Rangordnung einen im Vergleich zu den übrigen aktiven Geräten 2A-2N, von denen die Fußsteuerung 1 einen Identifikationscode empfangen hat, priorisierten individuellen Rang einnimmt oder Wert aufweist.

Senden beispielsweise die Geräten 2A, 2B ihre spezifischen Identifikationscodes "WHTW" und "WHUL" und ist jenes Gerät priorisiert, dessen Rang oder Wert in der Rangordnung niedriger ist, so wählt die Steuerlogik oder Fußsteuerung 1 das Gerät 2A mit dem Identifikationscode "WHTW" und dem Rang "0" zur drahtlosen Übertragung von Betriebsdaten aus.

Im Folgenden wird das Ausführungsbeispiel gemäß den Figuren 4 - 5 weiter erläutert. Die Figur 5 zeigt schematisch die Speicherelemente 13 der Geräte 6A-6N (in den Geräten 6A-6N entsprechender Reihung), wobei in jedem der Speicherelemente 13 ein spezifisches Datenpaket 5, 5A-5N gespeichert ist. Jedes der Datenpakete 5A-5N umfasst einen spezifischen Identifikationscode (WHTW, WHUL, WHST, WHNN) der durch die Fußsteuerung 1 ansteuerbaren Geräte 6A-6N und einen jedem Gerät 6A-6N zugeordneten, individuellen, eindeutigen Rang einer Rangordnung der durch die Fußsteuerung 1 ansteuerbaren Geräte 6A-6N. Die Ränge oder die Werte der Ränge sind beispielhaft mit "A0", "B0", "C0" und "D0" bezeichnet, wobei dem Gerät 6A mit dem Identifikationscode "WHTW" der Rang "A0", dem Gerät 6B mit dem Identifikationscode "WHUL" der Rang "B0", dem Gerät 6C mit dem Identifikationscode "WHST" der Rang "C0" und dem Gerät 6N mit dem Identifikationscode "WHNN" der Rang "D0" zugeordnet ist. Somit ist jedem der durch die Fußsteuerung 1 ansteuerbaren Gerät 2A-2N ein eigenes, spezifisches Datenpaket 4 mit einem spezifischen Identifikationscode, der insbesondere eindeutig ist und von jedem Identifikationscode der anderen Geräte 2A-2N unterscheidbar ist, und ein individueller, eindeutiger Rang zugeordnet.

Die Auswahl an welches der durch die Fußsteuerung 1 ansteuerbaren Gerät 6A-6N drahtlos Betriebsdaten oder Steuerungsdaten übertragen werden geschieht gemäß dem Ausführungsbeispiel der Figuren 4-5 wie folgt: damit Betriebsdaten an ein Gerät 6A-6N übertragen werden können, muss das Gerät 6A-6N aktiv sein oder sich in einem Betriebsmodus befinden, in dem es insbesondere zum drahtlosen Senden und Empfangen von Daten bereit ist. Das Aktivieren des Geräts 6A-6N oder das in den Betriebsmodus Versetzen kann beispielsweise durch den Anwender bewirkt werden, in dem dieser das Gerät 6A-6N ergreift, bewegt und/ oder ein Stellelement betätigt. Im Folgenden sendet ein aktives Gerät 6A-6N sein jeweiliges, spezifisches Datenpaket 5 drahtlos über die Sende- und Empfangseinheit 9A an die Fußsteuerung 1.

Sind mehrere Geräte 6A-6N aktiv oder senden (innerhalb eines vorbestimmten Zeitraums) mehrere Geräte 6A-6N ihren jeweils spezifisches Datenpaket 5, so empfängt die Fußsteuerung 1 diese spezifischen Datenpaket 5 und leitet diese an die Steuerlogik 3 weiter. Die Steuerlogik 3 erkennt aufgrund der in den empfangenen Datenpaketen 5 enthaltenen Identifikationscodes welche Geräte 6A-6N aktiv sind und kann deren individuelle Ränge oder die Werte der Ränge bestimmen. In Abhängigkeit davon welcher individuelle Rang der aktiven Geräte 6A-6N priorisiert ist, wählt die Steuerlogik oder Fußsteuerung 1 jenes Gerät 6A-6N zur ausschließlichen drahtlosen Übertragung von Betriebsdaten, insbesondere Steuerungsdaten für die Behandlung und/ oder Diagnose, aus, von dem die Fußsteuerung 1 ein Datenpaket 5 empfangen hat und das in der Rangordnung einen im Vergleich zu den übrigen aktiven Geräten 6A-6N, von denen die Fußsteuerung 1 ein Datenpaket 5 empfangen hat, priorisierten individuellen Rang einnimmt oder Wert aufweist.

Senden beispielsweise die Geräten 6B und 6C ihre spezifischen Datenpakete 5B (mit dem Identifikationscode "WHUL" und dem Rang "B0") und 5C (mit dem Identifikationscode "WHST" und dem Rang "C0") und ist jenes Gerät priorisiert, dessen Rang den im Alphabet vorgereihten Buchstaben aufweist, so wählt die Steuerlogik oder Fußsteuerung 1 das Gerät 6B mit dem Identifikationscode "WHUL" und dem Rang "B0" zur drahtlosen Übertragung von Betriebsdaten aus.

Für beide Ausführungsbeispiele gemäß den Figuren 1-3 und 4-5 gilt, dass, wenn nur ein einziges der Geräte 2A-2N, 6A-6N aktiv ist oder (innerhalb eines vorbestimmten Zeitraums) einen Identifizierungscode sendet, so wählt die Fußsteuerung dieses Gerät 2A-2N, 6A-6N aus und sendet ausschließlich an dieses Gerät 2A-2N, 6A-6N Betriebsdaten oder Steuerungsdaten für die Behandlung und/ oder Diagnose. Vorzugsweise ist die Steuerlogik hierbei so ausgebildet, dass das Ermitteln des individuellen Rangs des einzig aktiven Geräts 2A-2N, 6A-6N oder der Vergleich von individuellen Rängen nicht durchgeführt oder übersprungen wird.

Das in der Figur 6 dargestellte medizinischen oder dentalen System 100 ist als Prophylaxe-Tischgerät ausgebildet. Das System 100 weist eine Fußsteuerung 1 auf, die im Aufbau den Fußsteuerungen der Figuren 1 - 5 entspricht. Das System 100 umfasst des Weiteren ein Gerät oder Handstück 2 zum rotierenden Antrieb eines Werkzeugs, zum Beispiel einer Prophy-Kappe oder einer Bürste, und ein Gerät 6 mit einem Schwingantrieb, insbesondere einem Ultraschallantrieb, zum schwingenden Antrieb eines Werkzeugs. Beide Geräte 2, 6 sind ausgebildet, drahtlos (siehe Pfeile 10) mit der Fußsteuerung 1 zu kommunizieren und insbesondere Betriebsdaten oder Steuerungsdaten für die Behandlung von der Fußsteuerung 1 zu erhalten.

Das Gerät 6 umfasst ein Handstück 14, insbesondere ein Scalerhandstück zur Entfernung von Zahnstein, das über ein Kabel 15 mit einer Versorgungs- und/ oder Steuereinheit 16 verbunden ist. Vorzugsweise ist die Sende- und Empfangseinheit 9A des Geräts 6 in der Versorgungs- und/ oder Steuereinheit 16 vorgesehen. Die Versorgungs- und/ oder Steuereinheit 16 umfasst eine Steuerung zum Steuern des Betriebs des Handstücks 14, ein Stellelement 18 zum Stellen oder Auswählen von Betriebsparametern oder -modi und zumindest einen Behälter 17 für ein Behandlungsmedium. Das Behandlungsmedium und Steuersignale werden dem Handstück 14 über das Kabel 15 zugeleitet.

Die Auswahl, an welches der durch die Fußsteuerung 1 ansteuerbaren Gerät 2, 6 drahtlos Betriebsdaten oder Steuerungsdaten übertragen werden, kann bei dem medizinischen oder dentalen System 100 gemäß dem Ausführungsbeispiel der Figuren 1-3 oder der Figuren 4-5 realisiert sein. Dem entsprechend sind die Datenpakete 4, 5 entweder in der Fußsteuerung 1 oder in den Geräten 2, 6 vorgesehen und das System 100 wird entweder gemäß dem im Vorstehenden beschriebenen Verfahren nach den Figuren 1-3 oder den Figuren 4-5 betrieben.

Bei dem medizinischen oder dentalen System 100 ist des Weiteren vorgesehen, dass die individuellen Ränge der Geräte 2, 6 in den Datenpaketen 4 oder 5 durch das Pairen der Fußsteuerung 1 mit den Geräten 2, 6 bestimmt werden. Gemäß dem System 100 wird das Gerät 2 kabellos mit der Fußsteuerung 1 gepairt, während das Gerät 6 über ein Kabel 19 mit der Fußsteuerung 1 gepairt wird (siehe Figur 7). Für das Pairing mit oder ohne Kabel werden unterschiedliche Treiber benötigt, die vorzugsweise in der Fußsteuerung 1 gespeichert sind. Die Steuereinheiten der Geräte 2, 6 und/ oder die Steuerung oder der Mikrocontroller 7 der Fußsteuerung 1 sind ausgebildet, die unterschiedlichen Treiber und vorzugsweise aufgrund der unterschiedlichen Treiber die Art des Pairings zu erkennen und dem Gerät 2, 6, das gerade mit der Fußsteuerung gepairt wird, einen dem Treiber und/ oder der Art des Parings entsprechenden Rang zuzuordnen. Insbesondere sind die Steuereinheiten der Geräte 2, 6 und/ oder die Steuerung oder der Mikrocontroller 7 der Fußsteuerung 1 ausgebildet, den Rang dem Identifikationscode des Geräts 2, 6, das gerade mit der Fußsteuerung gepairt wird, zuzuordnen, um damit die Datenpakete 4, 5 zu bilden, oder den Rang in dem Datenpaket 4, 5 des jeweiligen Gerät 2, 6 einzufügen.

Die beschriebenen oder dargestellten Ausführungsbeispiele dienen insbesondere der Veranschaulichung der Erfindung.

## Patentansprüche

1. Verfahren zur drahtlosen Übertragung von Daten in einem medizinischen oder dentalen System (50, 70, 100), das eine Fußsteuerung (1) und mehrere medizinische oder dentale Geräte (2, 2A-2N; 6, 6A-6N) zur Behandlung und/ oder Diagnose umfasst, wobei zumindest zwei der mehreren Geräte (2, 2A-2N; 6, 6A-6N) wahlweise durch die Fußsteuerung (1) drahtlos ansteuerbar sind und wobei jedem durch die Fußsteuerung (1) ansteuerbaren Gerät (2, 2A-2N; 6, 6A-6N) ein spezifischer Identifikationscode zugeordnet ist, wobei das Verfahren **dadurch gekennzeichnet ist, dass**
eine in der Fußsteuerung (1) vorgesehenen Steuerlogik (3) auf Basis von Datenpaketen (4, 4A-4N; 5, 5A-5N) jenes durch die Fußsteuerung (1) ansteuerbare Gerät (2, 2A-2N; 6, 6A-6N) auswählt, an das nachfolgend drahtlos Betriebsdaten zum Betrieb des Geräts (2, 2A-2N; 6, 6A-6N), insbesondere Steuerungsdaten für die Behandlung und/ oder Diagnose, übertragen werden, wobei jedem durch die Fußsteuerung (1) ansteuerbaren Gerät (2, 2A-2N; 6, 6A-6N) ein eigenes Datenpaket (4, 4A-4N; 5, 5A-5N) zugeordnet ist und wobei jedes dieser Datenpakete (4, 4A-4N; 5, 5A-5N) den spezifischen Identifikationscode des durch die Fußsteuerung (1) ansteuerbaren Geräts (2, 2A-2N; 6, 6A-6N), dem das Datenpaket (4, 4A-4N; 5, 5A-5N) zugeordnet ist, und einen diesem Gerät (2, 2A-2N; 6, 6A-6N) zugeordneten, individuellen Rang einer Rangordnung der durch die Fußsteuerung (1) ansteuerbaren Geräte (2, 2A-2N; 6, 6A-6N) umfasst, wobei
die Fußsteuerung (1) nur jenes Gerät (2, 2A-2N; 6, 6A-6N) zur drahtlosen Übertragung von Betriebsdaten für die Behandlung und/ oder Diagnose auswählt und nur zu jenem Gerät (2, 2A-2N; 6, 6A-6N) eine drahtlose Kommunikationsverbindung zur Übertragung von Betriebsdaten für die Behandlung und/ oder Diagnose aufbaut, von dem die Fußsteuerung (1) einen Identifikationscode empfangen hat und das in der Rangordnung im Vergleich zu den übrigen Geräten (2, 2A-2N; 6, 6A-6N), von denen die Fußsteuerung (1) einen Identifikationscode empfangen hat, einen priorisierten individuellen Rang aufweist.

2. Verfahren zur drahtlosen Übertragung von Daten nach Anspruch 1, **dadurch gekennzeichnet, dass**
die Datenpakete (4, 4A-4N; 5, 5A-5N) der durch die Fußsteuerung (1) ansteuerbaren Geräte (2, 2A-2N; 6, 6A-6N) in der Fußsteuerung (1) vorgesehen sind.

3. Verfahren zur drahtlosen Übertragung von Daten nach Anspruch 2, **dadurch gekennzeichnet, dass**
eines oder mehrere der durch die Fußsteuerung (1) ansteuerbaren Geräte (2, 2A-2N; 6, 6A-6N) ihren jeweiligen im Gerät (2, 2A-2N; 6, 6A-6N) gespeicherten, spezifischen Identifikationscode an die Fußsteuerung (1) senden, die Fußsteuerung (1) diese(n) spezifischen Identifikationscode(s) empfängt und die Steuerlogik (3) auf Basis des oder der empfangenen Identifikationscode(s) und der Datenpakete (4, 4A-4N; 5, 5A-5N) erkennt, welche(s) der durch die Fußsteuerung (1) ansteuerbaren Geräte (2, 2A-2N; 6, 6A-6N) aktiv ist/ sind und welchen individuellen Rang das oder die aktive(n) Gerät(e) (2, 2A-2N; 6, 6A-6N) in der Rangordnung einnimmt/ einnehmen.

4. Verfahren zur drahtlosen Übertragung von Daten nach Anspruch 1, **dadurch gekennzeichnet, dass**
jedes durch die Fußsteuerung (1) ansteuerbare Gerät (2, 2A-2N; 6, 6A-6N) sein ihm zugeordnetes Datenpaket (4, 4A-4N; 5, 5A-5N) aufweist.

5. Verfahren zur drahtlosen Übertragung von Daten nach Anspruch 4, **dadurch gekennzeichnet, dass**
wenn die Fußsteuerung (1) Datenpakete (4, 4A-4N; 5, 5A-5N) von zwei oder mehr durch die Fußsteuerung (1) ansteuerbaren Geräten (2, 2A-2N; 6, 6A-6N) empfängt, dann wählt die Fußsteuerung (1) jenes Gerät (2, 2A-2N; 6, 6A-6N) zur drahtlosen Übertragung von Betriebsdaten aus, von dem die Fußsteuerung (1) ein Datenpaket (4, 4A-4N; 5, 5A-5N) empfangen hat und dessen individueller Rang einen im Vergleich zu den übrigen Geräten (2, 2A-2N; 6, 6A-6N), von denen die Fußsteuerung (1) ein Datenpaket (4, 4A-4N; 5, 5A-5N) empfangen hat, priorisierten individuellen Rang einnimmt oder Wert aufweist.

6. Verfahren zur drahtlosen Übertragung von Daten nach einem der vorstehenden Ansprüche, **dadurch gekennzeichnet, dass**
wenn die Fußsteuerung (1) einen Identifikationscode oder ein Datenpaket (4, 4A-4N; 5, 5A-5N) nur von einem einzigen durch die Fußsteuerung (1) ansteuerbaren Geräte (2, 2A-2N; 6, 6A-6N) empfängt, dann wählt die Steuerlogik (3) der Fußsteuerung (1) dieses Gerät (2, 2A-2N; 6, 6A-6N) zur drahtlosen Übertragung von Betriebsdaten aus.

7. Verfahren zur drahtlosen Übertragung von Daten nach einem der vorstehenden Ansprüche, **dadurch gekennzeichnet, dass**
die individuellen Ränge der Datenpakete (4, 4A-4N; 5, 5A-5N) durch das Pairen und/ oder während des Pairens der durch die Fußsteuerung (1) ansteuerbaren Geräte (2, 2A-2N; 6, 6A-6N) mit der Fußsteuerung (1) bestimmt werden.

8. Verfahren zur drahtlosen Übertragung von Daten nach Anspruch 7, **dadurch gekennzeichnet, dass**
die individuellen Ränge durch die Reihenfolge, in welcher die durch die Fußsteuerung (1) ansteuerbaren Geräte (2, 2A-2N; 6, 6A-6N) mit der Fußsteuerung (1) gepairt werden, bestimmt werden.

9. Verfahren zur drahtlosen Übertragung von Daten nach Anspruch 7 oder 8, **dadurch gekennzeichnet, dass**
die individuellen Ränge durch den für das Pairen verwendeten Treiber und/ oder die Art des Pairings zwischen der Fußsteuerung (1) und der durch die Fußsteuerung (1) ansteuerbaren Geräte (2, 2A-2N; 6, 6A-6N) bestimmt werden, wobei die Art des Pairings zum Beispiel eine drahtgebundene Verbindung und eine drahtlose Verbindung umfasst.

10. Verfahren zur drahtlosen Übertragung von Daten nach einem der vorstehenden Ansprüche, **dadurch gekennzeichnet, dass**
jeder individuelle Rang eines Geräts (2, 2A-2N; 6, 6A-6N) in der Rangordnung nur einmal vorhanden ist.

11. Computerprogrammprodukt oder computerlesbares Speichermedium, umfassend Befehle, die bei der Ausführung durch einen Computer (7) diesen veranlassen, das Verfahren nach einem der vorstehenden Ansprüche 1 - 10 auszuführen.

12. Fußsteuerung (1) zur wahlweisen drahtlosen Steuerung eines von zumindest zwei durch die Fußsteuerung (1) ansteuerbaren, medizinischen oder dentalen Geräten (2, 2A-2N; 6, 6A-6N), umfassend: zumindest ein Stellelement (8) zum Stellen eines Wertes von Betriebsdaten für die zumindest zwei Geräte (2, 2A-2N; 6, 6A-6N) und eine Sende- und Empfangseinheit (9) zur bidirektionalen, drahtlosen Kommunikation mit den zumindest zwei Geräten (2, 2A-2N; 6, 6A-6N) einschließlich der Übertragung der durch das Stellelement (8) stellbaren Betriebsdaten, **gekennzeichnet durch** eine Vorrichtung zur Datenverarbeitung (7) mit Mitteln zur Ausführung des Verfahrens nach einem der vorstehenden Ansprüche 1 - 10.

13. Fußsteuerung (1) nach Anspruch 12, **dadurch gekennzeichnet, dass** die Vorrichtung zur Datenverarbeitung (7) die Steuerlogik (3) zur Auswahl jenes Geräts (2, 2A-2N; 6, 6A-6N), an welches die Fußsteuerung (1) drahtlos Betriebsdaten für die Behandlung und/ oder Diagnose überträgt, umfasst.

14. Medizinisches oder dentales System (50, 70, 100), **gekennzeichnet durch** eine Fußsteuerung (1) nach Anspruch 12 oder 13 und mehrere medizinische oder dentale Geräte (2, 2A-2N; 6, 6A-6N) zur Behandlung und/ oder Diagnose, wobei zumindest zwei der mehreren Geräte (2, 2A-2N; 6, 6A-6N) durch die Fußsteuerung (1) ansteuerbar sind und die Fußsteuerung (1) ausgebildet ist, wahlweise eines der zumindest zwei Geräte (2, 2A-2N; 6, 6A-6N) durch die Fußsteuerung (1) anzusteuern.

15. Medizinisches oder dentales System (50, 70, 100) nach Anspruch 14, **dadurch gekennzeichnet, dass**
zumindest eines der durch die Fußsteuerung (1) ansteuerbaren Geräte (2, 2A-2N; 6, 6A-6N) ein dentales Prophylaxe-Gerät (2, 2A-2N; 6, 6A-6N) umfasst, zum Beispiel ein Gerät (2, 2A-2N; 6, 6A-6N) zur Entfernung von Zahnstein oder Plaque oder Verfärbungen oder ein Gerät (2, 2A-2N; 6, 6A-6N) zur Abgabe eines Pulvers auf eine Behandlungsstelle.

## Claims

1. Method for wireless transmission of data in a medical or dental system (50, 70, 100) comprising a foot control (1) and a plurality of medical or dental devices (2, 2A-2N; 6, 6A-6N) for treatment and/or diagnosis, wherein at least two of the plurality of devices (2, 2A-2N; 6, 6A-6N) are selectively wirelessly controllable by the foot control (1), and wherein each device (2, 2A-2N; 6, 6A-6N) controllable by the foot control (1) is assigned a specific identification code, the method being **characterized in that**
a control logic (3) provided in the foot control (1) selects, on the basis of data packets (4, 4A-4N; 5, 5A-5N), that device (2, 2A-2N; 6, 6A-6N) which can be actuated by the foot control (1) and to which subsequently operating data for operating device (2, 2A-2N; 6, 6A-6N), in particular control data for treatment and/or diagnosis, are transmitted wirelessly, wherein each device (2, 2A-2N; 6, 6A-6N) which can be actuated by the foot control (1) is assigned its own data packet (4, 4A-4N; 5, 5A-5N) and wherein each of these data packets (4, 4A-4N; 5, 5A-5N) comprises the specific identification code of the device (2, 2A-2N; 6, 6A-6N) controllable by the foot control (1) to which the data packet (4, 4A-4N; 5, 5A-5N) is assigned to, and an individual rank, assigned to this device (2, 2A-2N; 6, 6A-6N), of a ranking of the devices (2, 2A-2N; 6, 6A-6N) controllable by the foot control (1), wherein
the foot control (1) selects only that device (2, 2A-2N; 6, 6A-6N) for the wireless transmission of operating data for treatment and/or diagnosis and establishes a wireless communication link for the transmission of operating data for treatment and/or diagnosis only to that device (2, 2A-2N; 6, 6A-6N) from which the foot control (1) has received an identification code and which has a prioritised individual rank in the ranking compared to the other devices (2, 2A-2N; 6, 6A-6N) from which the foot control (1) has received an identification code.

2. The method for wireless transmission of data according to claim 1, **characterized in that**
the data packets (4, 4A-4N; 5, 5A-5N) of the devices (2, 2A-2N; 6, 6A-6N) controllable by the foot control (1) are provided in the foot control (1).

3. The method for wireless transmission of data according to claim 2, **characterized in that**
one or more of the devices (2, 2A-2N; 6, 6A-6N) controllable by the foot control (1) transmit their respective specific identification code stored in the device (2, 2A-2N; 6, 6A-6N) to the foot control (1), the foot control (1) receives this or these specific identification code(s), and the control logic (3) recognizes, on the basis of the received identification code(s) and the data packets (4, 4A-4N; 5, 5A-5N), which of the devices (2, 2A-2N; 6, 6A-6N) controllable by the foot control (1) is/are active and which individual rank the active device(s) (2, 2A-2N; 6, 6A-6N) has/ have in the ranking.

4. The method for wireless transmission of data according to claim 1, **characterized in that**
each device (2, 2A-2N; 6, 6A-6N) controllable by the foot control (1) comprises its associated data packet (4, 4A-4N; 5, 5A-5N).

5. The method for wireless transmission of data according to claim 5, **characterized in that**
when the foot control (1) receives data packets (4, 4A-4N; 5, 5A-5N) from two or more devices (2, 2A-2N; 6, 6A-6N) controllable by the foot control (1), then the foot control (1) selects that device (2, 2A-2N; 6, 6A-6N) for the wireless transmission of operating data from which the foot control (1) has received a data packet (4, 4A-4N; 5, 5A-5N) and whose individual rank has a prioritized individual rank or value in comparison with the other devices (2, 2A-2N; 6, 6A-6N) from which the foot control (1) has received a data packet (4, 4A-4N; 5, 5A-5N).

6. The method for wireless transmission of data according to any of the preceding claims, **characterized in that**
when the foot control (1) receives an identification code or a data packet (4, 4A-4N; 5, 5A-5N) only from a single device (2, 2A-2N; 6, 6A-6N) controllable by the foot control (1), then the control logic (3) of the foot control (1) selects this device (2, 2A-2N; 6, 6A-6N) for wireless transmission of operational data.

7. The method for wireless transmission of data according to one of the preceding claims, **characterized in that**
the individual ranks of the data packets (4, 4A-4N; 5, 5A-5N) are determined by pairing and/or during pairing of the devices (2, 2A-2N; 6, 6A-6N) controllable by the foot control (1) with the foot control (1).

8. The method for wireless transmission of data according to claim 7, **characterized in that**
the individual ranks are determined by the order in which the devices (2, 2A-2N; 6, 6A-6N) controllable by the foot control (1) are paired with the foot control (1).

9. The method for wireless transmission of data according to claim 7 or 8, **characterized in that**
the individual ranks are determined by the driver used for pairing and/or the type of pairing between the foot control (1) and the devices (2, 2A-2N; 6, 6A-6N) controllable by the foot control (1), wherein the type of pairing comprises, for example, a wired connection and a wireless connection.

10. The method for wireless transmission of data according to one of the preceding claims, **characterized in that**
in the ranking each individual rank of a device (2, 2A-2N; 6, 6A-6N) is present only once.

11. A computer program product or computer-readable storage medium comprising instructions which, when executed by a computer (7), cause the computer (7) to perform the method of any of the preceding claims 1-10.

12. A foot control (1) for selective wireless control of one of at least two medical or dental devices (2, 2A-2N; 6, 6A-6N) controllable by the foot control (1), comprising: at least one actuator (8) for setting a value of operating data for the at least two devices (2, 2A-2N; 6, 6A-6N) and a transmitting and receiving unit (9) for bidirectional wireless communication with the at least two devices (2, 2A-2N; 6, 6A-6N) including transmission of the operating data settable by the actuator (8), **characterized by** a device for data processing (7) with means for carrying out the method according to one of the preceding claims 1-10.

13. The foot control (1) according to claim 12, **characterized in that** the data processing device (7) comprises the control logic (3) for selecting that device (2, 2A-2N; 6, 6A-6N) to which the foot control (1) wirelessly transmits operating data for treatment and/or diagnosis.

14. A medical or dental system (50, 70, 100), **characterized by**
a foot control (1) according to claim 12 or 13 and a plurality of medical or dental devices (2, 2A-2N; 6, 6A-6N) for treatment and/or diagnosis, wherein at least two of said plurality of devices (2, 2A-2N; 6, 6A-6N) are controllable by said foot control (1) and said foot control (1) is configured to selectively control one of said at least two devices (2, 2A-2N; 6, 6A-6N).

15. The medical or dental system (50, 70, 100) according to claim 14, **characterized in that**
at least one of the devices (2, 2A-2N; 6, 6A-6N) controllable by the foot control (1) comprises a dental prophylaxis device (2, 2A-2N; 6, 6A-6N), for example a device (2, 2A-2N; 6, 6A-6N) for removing tartar or plaque or stains or a device (2, 2A-2N; 6, 6A-6N) for delivering a powder to a treatment site.

## Revendications

1. Procédé, destiné à la transmission sans fil de données dans un système (50, 70, 100) médical ou dentaire, qui comprend une commande au pied (1) et plusieurs instruments (2, 2A à 2N ; 6, 6A à 6N) médicaux ou dentaires, pour le traitement et / ou pour le diagnostic, au moins deux des plusieurs instruments (2, 2A à 2N ; 6, 6A à 6N) étant susceptibles d'être actionnés sans fil sélectivement par la commande au pied (1) et à chaque instrument (2, 2A à 2N ; 6, 6A à 6N) actionnable par la commande au pied (1) étant associé un code d'identification spécifique, le procédé étant **caractérisé**
**en ce qu'**une logique de commande (3) prévue dans la commande au pied (1) sélectionne sur la base de paquets de données (4, 4A à 4N ; 5, 5A à 5N) l'instrument (2, 2A à 2N ; 6, 6A à 6N) actionnable par la commande au pied (1) auquel sont transmis sans fil par la suite des données opérationnelles pour le fonctionnement de l'instrument (2, 2A à 2N ; 6, 6A à 6N), notamment des données de commande pour le traitement et / ou pour le diagnostic, à chaque instrument (2, 2A à 2N ; 6, 6A à 6N) actionnable par la commande au pied (1) étant associé un propre paquet de données (4, 4A à 4N ; 5, 5A à 5N) et chacun desdits paquets de données (4, 4A à 4N ; 5, 5A à 5N) comprenant le code d'identification spécifique de l'instrument (2, 2A à 2N ; 6, 6A à 6N) actionnable par la commande au pied (1), auquel le paquet de données (4, 4A à 4N ; 5, 5A à 5N) est associé et un rang individuel associé audit instrument (2, 2A à 2N ; 6, 6A à 6N) dans une hiérarchie des instruments (2, 2A à 2N ; 6, 6A à 6N) actionnables par la commande au pied (1),
la commande au pied (1) ne sélectionnant pour la transmission sans fil de données opérationnelles pour le traitement et / ou le diagnostic que ledit instrument (2, 2A à 2N ; 6, 6A à 6N) et n'établissant une connexion de communication sans fil pour la transmission de données que vers l'instrument (2, 2A à 2N ; 6, 6A à 6N) de la part duquel la commande au pied (1) a réceptionné un code d'identification et qui dans la hiérarchie présente un rang individuel priorisé en comparaison des autres instruments (2, 2A à 2N ; 6, 6A à 6N) de la part desquels la commande au pied (1) a réceptionné un code d'identification.

2. Procédé destiné à la transmission sans fil de données selon la revendication 1, **caractérisé**
**en ce que** les paquets de données (4, 4A à 4N ; 5, 5A à 5N) des instruments (2, 2A à 2N ; 6, 6A à 6N) actionnables par la commande au pied (1) sont prévus dans la commande au pied (1).

3. Procédé destiné à la transmission sans fil de données selon la revendication 2, **caractérisé**
**en ce qu'**un ou plusieurs des instruments (2, 2A à 2N ; 6, 6A à 6N) actionnables par la commande au pied (1) envoie(nt) à la commande au pied (1) leur code d'identification spécifique respectif mémorisé dans l'instrument (2, 2A à 2N ; 6, 6A à 6N), la commande au pied (1) réceptionne le(s)dit(s)codes d'identification spécifique(s) et la logique de commande (3) identifie sur la base du ou des code (s) d'identification réceptionné(s) et des paquets de données (4, 4A à 4N ; 5, 5A à 5N) le(s)quel(s) des instruments (2, 2A à 2N ; 6, 6A à 6N) actionnable(s) par la commande au pied (1) est / sont actif(s) et quel rang individuel occupe / occupent dans la hiérarchie le ou les instrument(s) (2, 2A à 2N ; 6, 6A à 6N) actifs.

4. Procédé destiné à la transmission sans fil de données selon la revendication 1, **caractérisé**
**en ce que** chaque instrument (2, 2A à 2N ; 6, 6A à 6N) actionnable par la commande au pied (1) comporte son paquet de données (4, 4A à 4N ; 5, 5A à 5N) qui lui est associé.

5. Procédé destiné à la transmission sans fil de données selon la revendication 4, **caractérisé**
**en ce que** lorsque la commande au pied (1) réceptionne des paquets de données (4, 4A à 4N ; 5, 5A à 5N) de la part de deux instruments (2, 2A à 2N ; 6, 6A à 6N) actionnables par la commande au pied (1) ou plus, alors la commande au pied (1) sélectionne pour la transmission sans fil de données opérationnelles l'instrument (2, 2A à 2N ; 6, 6A à 6N) de la part duquel la commande au pied (1) a réceptionné un paquet de données (4, 4A à 4N; 5, 5A à 5N) et dont le rang individuel occupe un rang individuel priorisé ou présente une valeur individuelle priorisée en comparaison des autres instruments (2, 2A à 2N ; 6, 6A à 6N) de la part desquels la commande au pied (1) a réceptionné un paquet de données (4, 4A à 4N ; 5, 5A à 5N).

6. Procédé destiné à la transmission sans fil de données selon l'une quelconque des revendications précédentes, **caractérisé**
**en ce que** lorsque la commande au pied (1) ne réceptionne un code d'identification ou un paquet de données (4, 4A à 4N ; 5, 5A à 5N) que de la part d'un seul instrument (2, 2A à 2N ; 6, 6A à 6N) actionnable par la commande au pied (1), alors la logique de commande (3) de la commande au pied (1) sélectionne ledit instrument (2, 2A à 2N ; 6, 6A à 6N) pour la transmission sans fil de données opérationnelles.

7. Procédé destiné à la transmission sans fil de données selon l'une quelconque des revendications précédentes, **caractérisé**
**en ce que** les rangs individuels des paquets de données (4, 4A à 4N ; 5, 5A à 5N) sont déterminés par l'appariement et / ou pendant l'appariement des instruments (2, 2A à 2N ; 6, 6A à 6N) actionnables par la commande au pied (1) avec la commande au pied (1).

8. Procédé destiné à la transmission sans fil de données selon la revendication 7, **caractérisé**
**en ce que** les rangs individuels sont déterminés par l'ordre chronologique dans lequel les instruments (2, 2A à 2N ; 6, 6A à 6N) actionnables par la commande au pied (1) sont appariés avec la commande au pied (1).

9. Procédé destiné à la transmission sans fil de données selon la revendication 7 ou 8, **caractérisé**
**en ce que** les rangs individuels sont déterminés par le pilote utilisé pour l'appariement et / ou par le type d'appariement entre la commande au pied (1) et les instruments (2, 2A à 2N ; 6, 6A à 6N) actionnables par la commande au pied (1), le type de l'appariement comprenant par exemple une connexion filaire et une connexion sans fil.

10. Procédé destiné à la transmission sans fil de données selon l'une quelconque des revendications précédentes, **caractérisé**
**en ce que** chaque rang individuel d'un instrument (2, 2A à 2N ; 6, 6A à 6N) n'est présent qu'une seule fois dans la hiérarchie.

11. Produit de programme informatique ou support d'enregistrement lisible par ordinateur, comprenant des instructions qui lors de l'exécution par un ordinateur (7) incitent celui-ci à réaliser le procédé selon l'une quelconque des revendications 1 à 10 précédentes.

12. Commande au pied (1), destinée à commander sans fil sélectivement l'un parmi au moins deux instruments (2, 2A 2N ; 6, 6A à 6N) médicaux ou dentaires actionnables par la commande au pied (1), comprenant: au moins un élément de réglage (8), destiné à régler une valeur de données opérationnelles pour les au moins deux instruments (2, 2A à 2N ; 6, 6A à 6N) et une unité émettrice et réceptrice (9), destinée à la communication bidirectionnelle sans fil avec les au moins deux instruments (2, 2A à 2N ; 6, 6A à 6N), y compris à la transmission des données opérationnelles réglables par l'élément de réglage (8), **caractérisée par**
un dispositif de traitement de données (7) pourvu de moyens pour la réalisation du procédé selon l'une quelconque des revendications 1 à 10 précédentes.

13. Commande au pied (1) selon la revendication 12, **caractérisée en ce que** le dispositif de traitement de données (7) comprend la logique de commande (3) destinée à sélectionner l'instrument (2, 2A à 2N ; 6, 6A à 6N) auquel la commande au pied (1) transmet sans fil des données opérationnelles pour le traitement et / ou le diagnostic.

14. Système (50, 70, 100) médical ou dentaire, **caractérisé par**
une commande au pied (1) selon la revendication 12 ou 13 et plusieurs instruments (2, 2A à 2N ; 6, 6A à 6N) médicaux ou dentaires pour le traitement et / ou le diagnostic, au moins deux parmi les plusieurs instruments (2, 2A à 2N ; 6, 6A à 6N) étant actionnables par la commande au pied (1) et la commande au pied (1) étant conçue pour actionner sélectivement l'un des au moins deux instruments (2, 2A à 2N ; 6, 6A à 6N) par la commande au pied (1).

15. Système (50, 70, 100) médical ou dentaire selon la revendication 14, **caractérisé en ce que** moins l'un des instruments (2, 2A à 2N ; 6, 6A à 6N) actionnables par la commande au pied (1) comprend un instrument de prophylaxie (2, 2A à 2N ; 6, 6A à 6N) dentaire, par exemple un instrument (2, 2A à 2N ; 6, 6A à 6N) pour éliminer le tartre ou la plaque dentaire ou des taches ou un instrument (2, 2A à 2N ; 6, 6A à 6N) de distribution d'une poudre sur une zone à traiter.
